# EUROPEAN PATENT SPECIFICATION

(11) **EP 0 265 193 B1**
(45) Date of publication and mention of the grant of the patent: **27.07.1994**
(21) Application number: 87309189.6
(22) Date of filing: 19.10.1987
(51) Int. Cl.: A61B 17/34

(54) **A trocar**
Trokar
Trocart

(30) Priority: 17.10.1986 US 920509
(43) Date of publication of application: 27.04.1988
(60) Divisional application: 93119774.3
(73) Proprietor: United States Surgical Corporation, Norwalk, Connecticut 06856 (US)
(72) Inventor: Lander, Jack R., Danbury CT 06811 (US); William A Holmes, Marblehead, MA 01945 (US); Peter F Costa, Sommerville, Mass 02143 (US)
(74) Representative: Marsh, Roy David

(56) References cited:
- DE-C- 103 373
- GB-A- 1 356 386
- US-A- 2 187 372
- US-A- 4 418 707
- US-A- 4 601 710
- US-A- 4 654 030

## Description

This invention relates to a trocar. More particularly, this invention relates to a latch means for a trocar.

As is known, a trocar is generally made of a stylet having a sharp tip for penetrating through an abdominal wall and a surrounding tube through which gas or the like may be injected or exhausted from the abdomen after puncturing by the stylet. Further, in order to protect a patient or surgeon from injury through inadertant contact with the sharp tip, protective tubes have been positioned over the stylet tip, for example, as described in U.S. Patent 4,601,710. In this case, the stylet is secured to a housing with a spring-biased protective tube concentrically about the stylet. When put to use, the protective tube is able to move proximally to expose the sharp tip for penetrating the abdominal wall without interference. Once a puncture has been made, the protective tube is able to slide distally under the force of the spring to again cover over the sharp tip to preclude injury to tissue within the abdomen. Thereafter, the stylet can be removed from the abdomen.

In addition, the known trocar has been constructed so that the housing secured to the stylet can be fitted into and rotated relative to a second housing containing an outer tube through which the stylet and protective tube may pass. Further, the second housing has had a flap valve which cooperates with a groove in the protective tube in order to lock the spring-biased protective tube in an extended position over the sharp stylet tip. Generally, the trocar is delivered for use in this locked condition so as to protect the user from being injured by the sharp tip of the stylet. However, prior to use, the housing connected to the stylet must be pushed into an unlocked position so that the protective tube can retract from the sharp tip in order to puncture an abdominal wall.

In the event that the trocar is not delivered in the locked position, there is a risk that users may be damaged should the protective tube inadvertently be retracted to expose the sharp stylet tip. Further, any exposed tissue of a patient may be damaged should the protective tube be retracted inadvertently prior to proper positioning of the stylet for penetrating an abdominal wall.

Accordingly, it is an object of the invention to provide a trocar in which the risk of injury due to inadvertant retraction of a protective tube over a sharp stylet tip can be significantly reduced.

It is another object of the invention to shield the sharp tip of a stylet of a trocar in a positive manner at all times prior to actual use.

It is another object to the invention to provide a relatively simple structure for shielding the sharp tip of a trocar until ready for use.

It is another object to the invention to provide a relatively simple mechanism for unlocking a trocar for use.

Briefly, the invention provides a trocar of two part construction.

One part of the trocar is formed of a housing, a stylet which is secured to and which extends from the housing with a sharp tip at a distal end, a tube which is slidably mounted in the housing between an extended position and a retracted position with the tube disposed about the stylet and a spring in the housing for biasing the tube outwardly of the housing into the extended position. In accordance with the invention, a latch means is provided in the housing for locking the tube in the extended position and a slider is slidably mounted in and projects from the housing for releasing the latch means to permit movement of the tube into the retracted position.

The second part of the trocar comprises a second housing which is coaxial of the first housing to define a handgrip and a guide tube which is secured to and which extends from the housing with the protective tube slidably mounted therein. The housing of the second part is also provided, in known manner, with a seal ring through which the protective tube of the first part slides in seal-tight manner and a flap valve which covers over the seal ring when the first part has been removed from the second part of the trocar.

When in use, the two housings are disposed in face-to-face manner with the slider of the first housing projecting against the second housing. In this position, the protective tube is retained in a locked position by the latch means. However, when the user is ready to penetrate, for example through an abdominal wall of the patient, the two housings are squeezed together so as to push the slider into the first housing to release the latch means. At this time, the protective tube is unlatched so as to be slid proximally during penetration through an abdominal wall as is known. However, once penetration has been completed, the protective tube is spring-biased distally into the extended position and is re-engaged by the latch means so as to be locked in place. The stylet may then be removed from the abdomen while the protective tube remains in a locked protective position about the sharp tip of the stylet.

The latch means includes a leaf spring which abuts the protective tube in the extended position as well as a spring finger which extends from the leaf spring into abutment with the slider for lifting of the leaf spring from the protective tube in response to movement of the slider into the housing. In this regard, a collar is provided on the protective tube to abut the leaf spring when in the extended position. In addition, the collar is slidable along the leaf spring in order to lift and release the spring finger from the slider at an intermediate point of movement along the leaf spring. Thus, once the slider has released the latch means, and the protective tube has moved a certain distance, the slider is no longer active so that any return of the protective tube to the extended position automatically activates the latch means into the locked position preventing further proximal movement of the protective tube.

An indicator may also be secured to the protective tube for exposure through the housing of the first part to indicate the position of the protective tube relative to the stylet tip during use.

These and other objects and advantages of the invention will become more apparent from the following description taken in conjunction with the accompanying drawings wherein:
Fig. 1 illustrates a perspective view of a trocar constructed in accordance with the invention.
Fig. 2 illustrates a cross-sectional view of the trocar of Fig. 1 in a locked position;
Fig. 3 illustrates a perspective view of a latch constructed in accordance with the invention;
Fig. 4 illustrates a partial cross-sectional view of the trocar in a position in which the protective tube has been initially released for penetration of an abdominal wall;
Fig. 5 illustrates a view similar to Fig. 4 with the spring finger released from the slider;
Fig. 6 illustrates a view similar to Figs. 4 and 5 with the latch means returned to a locked position with the protective tube in an extended position; and
Fig. 7 illustrates a part perspective view of the latch means and slider of the trocar in a locked position.

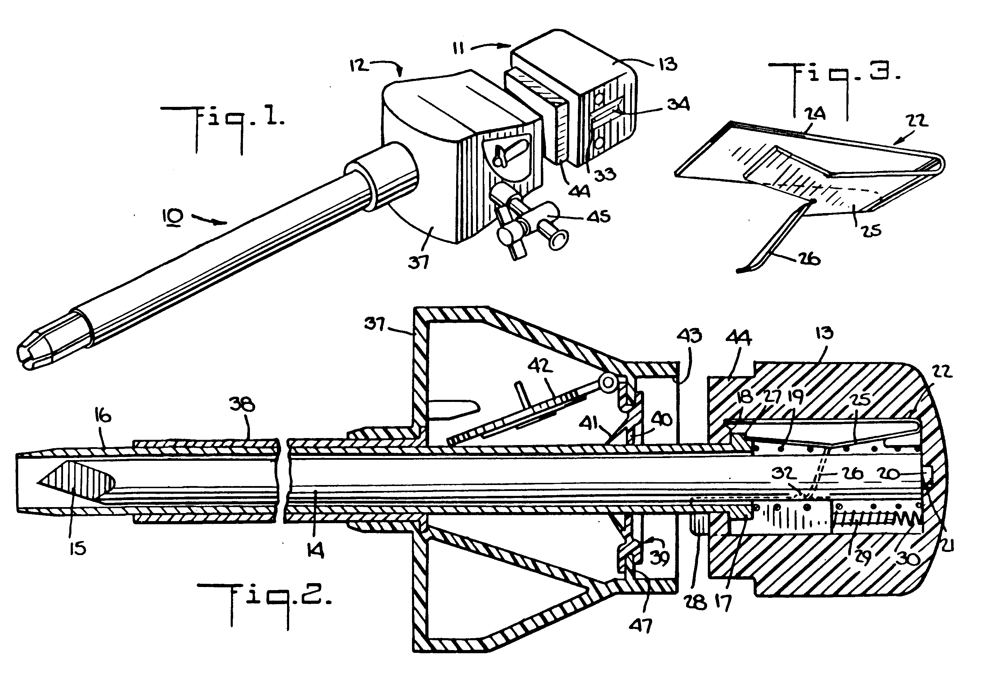

Referring to Fig. 1, the trocar 10 is constructed of two parts 11, 12 which are arranged in a coaxial manner.

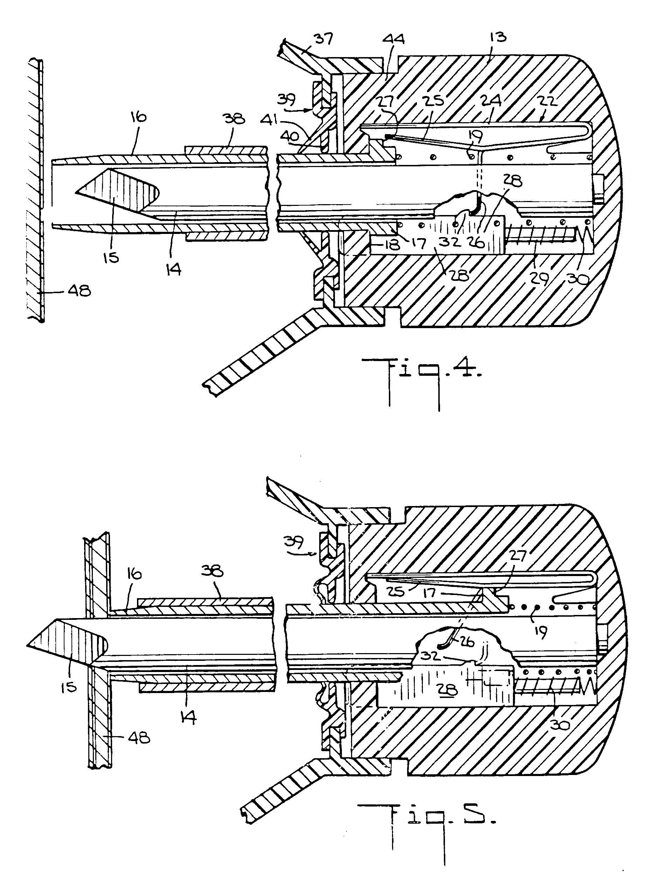

Referring to Figs. 1 and 2, one part 11 includes a housing 13, for example made of plastic and a stylet 14, for example made of steel, which is secured to and extends from the housing 13 with a sharp tip 15, as is known, at the distal end. In addition, a protective tube 16, for example made of steel or aluminum is slidably mounted in the housing 13 between an extended position, as viewed in Fig. 2, and a retracted position while being disposed concentrically about the stylet 14. The tube 16 carries a collar 17 at the proximal end within the housing 13 to abut against a wall 18 of the housing under the bias of a coil spring 19. As indicated, the spring 19 is concentric to the proximal end of the stylet 14 and abuts against a back wall of the housing 13. In addition, the stylet 14 has a reduced portion 20 which is disposed within a recess 21 in the back wall of the housing 13 and secured in place, for example by an adhesive or other suitable means.

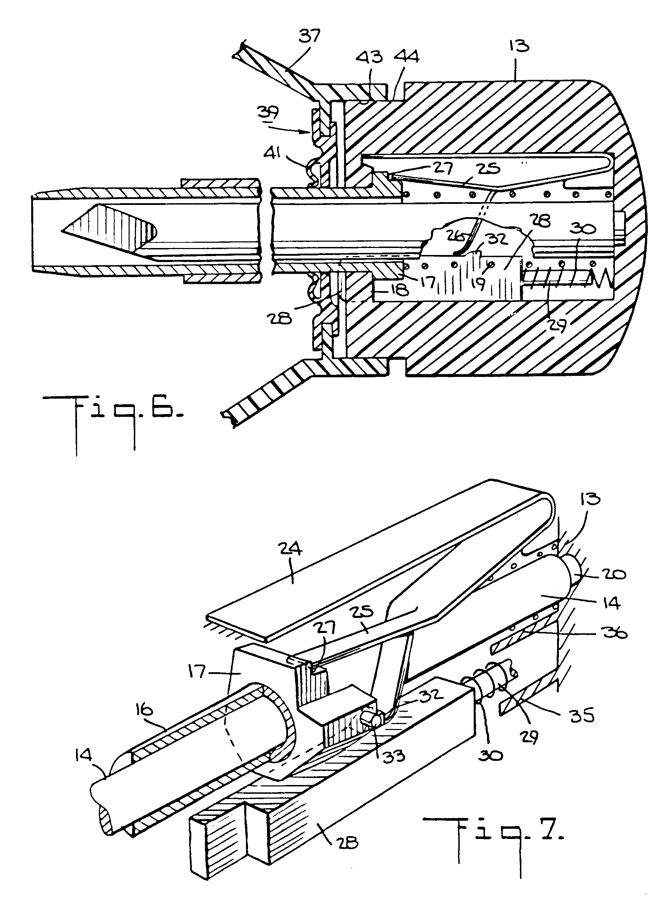

Referring to Figs. 2 and 3, a latch means 22 is disposed in the housing 13 for locking the protective tube 16 in the extended position illustrated. As indicated in Fig. 3, the latch means 22 is in the form of a one-piece body having a flat base 24, a leaf spring 25 which extends from one end of the base 24 to define a U-shape and a spring finger 26 which extends angularly and intermediately from the leaf spring 25. As shown, the leaf spring 24 has a proximal portion of a width equal to the width of the base 22 which extends from the base 22 to an intermediate point and a distal portion of reduced width extending to the distal end. The spring finger 26 extends from this intermediate point.

As indicated in Figs. 2 and 7, the reduced portion of the leaf spring 25 engages against the collar 17 on the protective tube 16. To this end, the collar 17 has a shoulder 27 at the upper end, as viewed, against which the end of the leaf spring 26 abuts. When the leaf spring 27 is in the position shown the protective sleeve 16 is prevented from moving proximally. As indicated in Fig. 2, the base 22 of the latch means is retained within the housing 13 in a fixed manner so as to be restrained against movement proximally or distally.

Referring to Fig. 2; a slider 28 is slidably mounted in and projects from the housing 13 for releasing the latch means 22 in order to permit movement of the protective tube 16 into a retracted position. The slider 28 is made of any suitable material, such as a metal or plastic and includes a stem 29 at the proximal end about which a spring 30 is disposed for biasing the slider 28 in a distal direction. As indicated, the spring 30 abuts against the back wall of the housing 13 while the slider has a shoulder portion 31 (see Fig. 7) for abutting against a front wall of the housing 13 in an extended position thereof. The slider 28 also has a shoulder 32 on an upper surface, as viewed, for engaging the free end of the spring finger 26. The shoulder 32 acts in the manner of a cam or single tooth ratchet so that when the slider 28 is moved into the housing 13 against the bias of the spring 30, the spring finger 26 pivots in a counter-clockwise manner as viewed while also lifting the leaf spring 25 from the collar 17 to release the protective tube 16 (see Fig. 4). Alternatively, other means such as a recess in the slider 28 can be used to accomplish the engagement of the spring finger 26. At this time, the tube 16 is freed to be able to move proximally, for example as indicated in Fig. 5. During this time, the collar 17 slides along the leaf spring 25 causing the leaf spring 25 to move towards the base 22 thereby lifting and releasing the spring finger 26 from the shoulder 32, for example at an intermediate point of movement of the collar 17 along the leaf spring 25.

Referring to Fig. 7, the collar 17 also carries a guide pin 33 which extends radially outwardly of the protective sleeve 16. As indicated in Fig. 1, this guide pin 33 is received in a guide slot 34 in a side wall of the housing 13. The guide pin 13 and slot 34 thus serve to retain the collar 17 in alignment with the leaf spring 25 during use. In addition, the pin 33 serves as an indicator to indicate the position of the protective tube 16 relative to the tip 15 of the stylet 14. For example, the distal most position (Fig. 1) indicates the tube 16 is in the extended position. A surgeon can thus determine where the tube 16 is during a surgical procedure.

Referring to Fig. 7, the housing 13 may be provided with a stop portion 35 for abutting the proximal end of the slider 28 and thus establish a rear most position of the slider 28. In similar manner, the housing may be provided with a stop portion 36 for abutting the collar 17 so as to provide a rear most position for the protective tube 16.

Referring to Figs. 1 and 2, the second part 12 of the trocar 10 is constructed in conventional manner. For example, this part 12 includes a housing 37, for example of plastic and a guide tube 38 which is secured to and extends from the housing 37 so as to slidably mount the protective tube therein. In addition, a seal ring 39 is mounted within the housing 37 about an opening through which the protective tube 16 of the trocar part 11 passes. This seal ring 39 includes a main portion 40 which sealingly engages the outer peripheral of the protective tube 16 and an annular flap 41 which extends from the main portion 40 to sealingly engage the protective tube 16 at a point distal from the main portion 40. During retraction of the protective tube 16, the seal flap 41 tends to deform in an S-shape manner as indicated in Fig. 5.

The housing 37 also has a flap valve 42 pivotally mounted therein in known manner for sealing over the seal ring 39 after the trocar part 11 has been removed.

Referring to Figs. 1 and 2, the housing 37 of the trocar part 12 is provided with a rectangular recess 43 at the proximal end while the housing 13 of the trocar part 11 is provided with a reduced portion 44 of rectangular shape which is sized to fit within the recess 43.

In addition, the housing 37 is provided with a valve construction 45 as well as a pointer to indicate the position of the flap valve 42.

In order to use the trocar 10, the two housing parts 11, 12 are placed together. For example, as indicated in Fig. 2, the protective tube 16 is slid through the ring seal 40 with the flap valve resting against the outer periphery of the protective tube 16. At this time, the leaf spring 25 engages against the shoulder 27 of the collar 17 so as to lock the protective tube 16 in the extended position. Also, the slider 28 projects out of the distal face of the housing 13.

Next, as indicated in Fig. 4, the two housings 13, 37 are brought together so that the reduced portion 44 of the housing 13 moves into the recess 43 of the housing 12 with the slider 28 abutting a rear wall 47 of the housing 37. Next, the user squeezes the two housings 13, 37 together so as to move the slider 28 proximally into the housing 13. In this regard, the two housings 13, 37 function as a handgrip for the user.

As indicated in Fig. 4, when the slider has moved into the housing 13, the spring finger 26 is pivoted in a counter-clockwise manner via the shoulder 32 on the slider 28 while also moving upwardly to lift the leaf spring 25 from the shoulder 27 of the collar 17. In the position shown, the protective tube 16 is free to move relative to the stylet 14. The user then penetrates through the abdominal wall 48 of a patient in known manner so that the sharp tip 15 of the stylet 14 punctures the wall 48 as indicated in Fig. 5. During this initial phase, the protective tube 16 moves proximally within the guide tube 38 with the collar 17 sliding along the leaf spring 25. At an intermediate point, for example where the finger 26 extends from the leaf spring 25, the collar deflects the leaf spring 25 upwardly causing the spring finger 26 to lift above the shoulder 32 on the slider 28. The spring finger 26 then pivots in a clockwise manner into the full line position illustrated in Fig. 5. The protective tube 16 may slide further proximally until abutting the stop 36 (see Fig. 7).

Of note, the stylet 14, protective tube 16 and guide tube 38 are sized so that the rear most retraction of the protective tube 16 places the distal end of the protective tube 16 slightly behind the sharp tip 15 of the stylet 14 but ahead of the guide tube 38. In addition, the respective diameters of the stylet 14 and tube 16, 38 are such that a slide fit relationship is presented so that both tubes 16, 38 may follow the sharp tip 15 through the wall 48 of the patient into the interior of the abdomen, as is known.

After the protective tube 16 passes through the final layer of the abdominal wall 48 and the sharp tip 15 is within an inflated cavity between the abdominal wall 48 and other organs, which cavity has been intentionally inflated, the spring 19 pushes the protective tube 16 outwardly to the extended position indicated in Fig. 6. At this time, the leaf spring 25 re-engages the shoulder 27 of the collar 17 to lock the protective tube 16 in place over the stylet tip 15. As indicated, the spring finger 26 is on the distal side of the shoulder 32 of the slider 28 and cannot be triggered again unless returned to the position indicated in Fig. 2.

The user may then release the housings 13, 37. Next, for example, the trocar part 11 may be slid out of the trocar part 12 with the flap valve 42 closing over the ring seal 40. This component 12 may then be used in a conventional manner. The removed component 11, on the other hand, has the guide tube 16 locked in place over the stylet tip 15 so as to protect against injury from the sharp edges of the tip 16.

In the event that the trocar 10 would require a second triggering of the latch means 22, the trocar part 11 can be slid slightly out of the housing 37 so that the slider 28 again projects out of the housing 13 so that the spring finger 26 engages behind the shoulder 32. A further squeezing action can then be carried out to again release the guide tube 16 in the manner described above.

The invention thus provides a trocar which can be easily manipulated by a user while the sharp tip of the stylet is maintained in a protective environment by the protective tube.

Further, the invention provides a trocar in which the protective tube is maintained in a locked position relative to the stylet tip until actual penetration through an abdominal wall or the like is required.

Of note, instead of using a latch means wherein a base and a leaf spring define a U-shape, the latch means may simply constitute a leaf spring mounted directly to the housing in a cantilevered manner with the spring finger depending therefrom.

## Claims

1. A trocar comprising
a first housing (13);
a stylet (14) secured to and extending from said housing, said stylet having a sharp tip (15) at a distal end;
a protective tube (16) slidably mounted in said housing between an extended position and a retracted position, said tube being disposed concentrically about said stylet;
a spring (19) in said housing biasing said tube outwardly of said housing into said extended position;
the trocar being characterised by:
latch means (22) in said housing for locking said tube in said extended position; and
a slider (28) mounted in and projecting from said housing for releasing said latch means to permit movement of said tube into said retracted position.

2. A trocar as claimed in claim 1 wherein said latch means includes a leaf spring (25) abutting said tube in said extended position and a spring finger (26) extending from said leaf spring into abutment with said slider for lifting said leaf spring from said tube in response to movement of said slider into said first housing.

3. A trocar as claimed in claim 2 which further comprises a collar (17) on said tube in abutment with said leaf spring in said extended position.

4. A trocar as claimed in claim 3 wherein said collar is slidable along said leaf spring to lift and release said spring finger from said slider at an intermediate point of movement along said leaf spring.

5. A trocar as claimed in claim 3 or 4, which further comprises a guide slot in said first housing and a guide pin secured to said collar and slidably mounted in said slot.

6. A trocar as claimed in any one of the preceding claims which further comprises a second housing (37) coaxial of said first housing (13) to define a handgrip therewith and a guide tube secured to and extending from said second housing with said protective tube slidably mounted therein.

7. A trocar as claimed in claim 6 which further comprises a seal ring (39) in said second housing having a main portion (40) sealingly engaging said protective tube and an annular flap (41) extending from said main portion and sealingly engaging said protective tube.

8. A trocar as claimed in claim 6 or 7 wherein said slider (28) projects from said first housing against said second housing whereby squeezing of said housings together pushes said slider into said first housing to release said latch means.

9. A trocar as claimed in any one of the preceding claims which further comprises a spring (30) biasing said slider outwardly of said first housing.

10. A trocar as claimed in any one of the preceding claims wherein said latch means is a unitary body having a base (24), a leaf spring (25) extending from said base to define a U-shape and a spring finger (26) extending from said leaf spring into abutment with said slider.

11. A trocar as claimed in claim 3 or any one of claims 4 to 10 as dependent on claim 3, wherein said spring finger (26) abuts means (32) on said slider, and said spring finger releases said leaf spring from said collar in response to proximal movement of said slider to permit proximal movement of said tube.

12. A trocar as claimed in claim 11 wherein said collar (17) is slidable along said leaf spring to lift and release said spring finger from said slider at an intermediate point of movement along said leaf spring.

## Patentansprüche

1. Ein Trokar mit
einem ersten Gehäuse (13),
einer Sonde (14), die am Gehäuse befestigt ist, und sich davon wegerstreckt, wobei die Sonde eine scharfe Spitze (15) an einem distalen Ende besitzt,
einem Schutzrohr (16), welches in dem Gehäuse zwischen einer ausgefahrenen Stellung und einer zurückgezogenen Stellung gleitbar angebracht ist, wobei das Rohr konzentrisch um die Sonde angeordnet ist,
einer Feder (19) in dem Gehäuse, welche das Rohr nach außen aus dem Gehäuse in die ausgefahrene Stellung vorspannt,
wobei das Trokar gekennzeichnet ist, durch Klinkeneinrichtungen (22) in dem Gehäuse zur Verriegelung des Rohrs in der ausgefahrenen Stellung und
einem Schieber (28), welcher im Gehäuse angebracht ist und aus dem Gehäuse hervorsteht, um die Klinkeneinrichtungen freizugeben, um eine Bewegung des Rohres in die zurückgezogene Stellung zu gestatten.

2. Trokar nach Anspruch 1,
wobei die Klinkeneinrichtung eine Blattfeder (25), welche in der ausgefahrenen Stellung mit dem Rohr in Anlage gerät, und einen Federfinger (26) aufweist, welcher sich von der Blattfeder weg in Anlage mit dem Schieber erstreckt, um die Blattfeder von dem Rohr als Folge einer Bewegung des Schiebers in das erste Gehäuse hinein anhebt.

3. Ein Trokar nach Anspruch 2,
welches weiterhin einen Kragen (17) am Rohr aufweist, welcher in der ausgefahrenen Stellung mit der Blattfeder in Anlage ist.

4. Ein Trokar nach Anspruch 3,
wobei der Kragen entlang der Blattfeder gleitbar ist, um den Federfinger an einem mittleren Bewegungspunkt entlang der Blattfeder vom Schieber anzuheben und freizugeben.

5. Ein Trokar nach Anspruch 3 oder 4,
welches weiterhin einen Führungsschlitz im ersten Gehäuse und einen an dem Kragen befestigten und gleitbar in dem Schlitz angebrachten Führungsstift aufweist.

6. Ein Trokar nach einem der vorhergehenden Ansprüche, welches koaxial zum ersten Gehäuse (13) weiterhin ein zweites Gehäuse (37) aufweist, um damit einen Handgriff zu bestimmen, und ein Führungsrohr besitzt, welches am zweiten Gehäuse befestigt ist, und daraus hervorsteht, wobei darin das Schutzrohr gleitbar angebracht ist.

7. Ein Trokar nach Anspruch 6,
welches weiterhin einen Dichtungsring (39) in dem zweiten Gehäuse aufweist, welcher einen Hauptabschnitt (40), der mit dem Schutzrohr dichtend im Eingriff steht, und eine ringförmige Klappe (41) besitzt, die sich vom Hauptabschnitt wegerstreckt, und mit dem Schutzrohr dichtend in Eingriff steht.

8. Ein Trokar nach Anspruch 6 oder 7,
wobei der Schieber (28) vom ersten Gehäuse gegen das zweite Gehäuse hervorsteht, wodurch ein Zusammendrücken der Gehäuse den Schieber in das erste Gehäuse hineindrückt, um die Klinkeneinrichtung freizugeben.

9. Ein Trokar nach einem der vorhergehenden Ansprüche,
welches weiterhin eine Feder (30) aufweist, die den Schieber nach außen aus dem ersten Gehäuse vorspannt.

10. Ein Trokar nach einem der vorhergehenden Ansprüche,
wobei die Klinkeneinrichtung ein einstückiger Körper ist, welcher ein Grundelement (24), eine Blattfeder (25), die sich vom Grundelement wegerstreckt, um eine U-Form zu bestimmen, und einen Federfinger (26) besitzt, welcher sich von der Blattfeder in Anlage mit dem Schieber wegerstreckt.

11. Ein Trokar nach Anspruch 3 oder einem der Ansprüche 4 bis 10 in Abhängigkeit von Anspruch 3,
wobei der Federfinger (26) mit Einrichtungen (32) am Schieber in Anlage gerät, und wobei der Federfinger die Blattfeder als Antwort auf eine proximale Bewegung des Schiebers vom Kragen freigibt, um eine proximale Bewegung des Rohrs zu gestatten.

12. Ein Trokar nach Anspruch 11,
wobei der Kragen (17) entlang der Blattfeder gleitbar ist, um den Federfinger vom Schieber an einem mittleren Bewegungspunkt entlang der Blattfeder anzuheben und freizugeben.

## Revendications

1. Trocart comprenant
un premier boîtier (13) ;
un stylet (14) fixé à et s'étendant dudit boîtier, ledit stylet présentant une pointe coupante (15) à une extrémité distale ;
un tube de protection (16) monté de façon coulissante dans ledit boîtier entre une position étendue et une position rétractée, ledit tube étant disposé concentriquement autour dudit stylet ;
un ressort (19) dans ledit logement sollicitant ledit tube vers l'extérieur dudit boîtier dans ladite position étendue ;
le trocart étant caractérisé par :
un moyen de verrouillage (22) dans ledit boîtier pour verrouiller ledit tube dans ladite position étendue; et
une coulisse (28) montée dans et faisant saillie dudit boîtier pour libérer ledit moyen de verrouillage pour permettre le déplacement dudit tube dans ladite position rétractée.

2. Trocart selon la revendication 1, dans lequel ledit moyen de verrouillage comprend un ressort à lames (25) butant contre ledit tube dans ladite position étendue et un doigt presseur (26) s'étendant dudit ressort à lames en butée avec ladite coulisse pour relever ledit ressort à lames dudit tube en réponse au déplacement de ladite coulisse dans ledit premier boîtier.

3. Trocart selon la revendication 2, qui comprend en outre un collier (17) sur ledit tube en butée avec ledit ressort à lames dans ladite position étendue.

4. Trocart selon la revendication 3, dans lequel ledit collier peut coulisser le long dudit ressort à lames pour relever et libérer ledit doigt presseur de ladite coulisse à un point intermédiaire de déplacement le long dudit ressort à lames.

5. Trocart selon les revendications 3 ou 4, qui comprend en outre une fente de guidage dans ledit premier boîtier et un axe de guidage fixé audit collier et monté de façon coulissante dans ladite fente.

6. Trocart selon l'une des revendications précédentes, qui comprend en outre un deuxième boîtier (37) coaxial audit premier boîtier (13) pour définir une poignée avec celui-ci et un tube de guidage fixé à et s'étendant depuis ledit deuxième boîtier, ledit tube de protection étant monté de façon coulissante à l'intérieur.

7. Trocart selon la revendication 6, qui comprend en outre un joint d'étanchéité (39) dans ledit deuxième boîtier présentant une partie principale (40) venant en prise de manière étanche avec ledit tube de protection et une patte annulaire (41) s'étendant depuis ladite partie principale et venant en prise de manière étanche avec ledit tube de protection.

8. Trocart selon la revendication 6 ou 7, dans lequel ladite coulisse (28) fait saillie dudit premier boîtier contre ledit deuxième boîtier par quoi la compression desdits boîtiers pousse ladite coulisse dans ledit premier boîtier afin de libérer ledit moyen de verrouillage.

9. Trocart selon l'une des revendications précédentes, qui comprend en outre un ressort (30) sollicitant ladite coulisse vers l'extérieur dudit premier boîtier.

10. Trocart selon l'une des revendications précédentes, dans lequel ledit moyen de verrouillage est un corps unitaire présentant une base (24), un ressort à lames (25) s'étendant depuis ladite base pour définir une forme en U et un doigt presseur (26) s'étendant dudit ressort à lames en butée avec ladite coulisse.

11. Trocart selon la revendication 3 ou l'une des revendications 4 à 10 dépendant de la revendication 3, dans lequel ledit doigt presseur (26) bute contre un moyen (32) sur ladite coulisse, et ledit doigt presseur libère ledit ressort à lames dudit collier en réponse à un déplacement proximal de ladite coulisse afin de permettre un mouvement proximal dudit tube.

12. Trocart selon la revendication 11, dans lequel ledit collier (17) peut coulisser le long dudit ressort à lames pour relever et libérer ledit doigt presseur de ladite coulisse à un point intermédiaire de déplacement le long dudit ressort à lames.
